**Europäisches Patentamt**

(19) **European Patent Office**

**Office européen des brevets**

(11) Veröffentlichungsnummer: **0 009 233**
**B1**

(12) **EUROPÄISCHE PATENTSCHRIFT**

(45) Veröffentlichungstag der Patentschrift:
**15.09.82**

(21) Anmeldenummer: **79103502.5**

(22) Anmeldetag: **18.09.79**

(51) Int. Cl.³: **A 61 M 1/00, A 61 M 27/00**

(54) **Saugflasche mit einem in ein Operationsfeld einlegbaren Drain.**

(30) Priorität: **20.09.78 DE 2840865**

(43) Veröffentlichungstag der Anmeldung:
**02.04.80 Patentblatt 80/7**

(45) Bekanntmachung des Hinweises auf die Patenterteilung:
**15.09.82 Patentblatt 82/37**

(84) Benannte Vertragsstaaten:
**AT BE FR GB NL**

(56) Entgegenhaltungen:
**DE-A-2 435 288**
**DE-U-7 336 232**
**DE-U-7 627 573**
**FR-A-2 304 360**
**US-A-3 809 087**

(73) Patentinhaber: **Sterimed Gesellschaft für medizinischen Bedarf mbH, Fasanerieweg 15, D-6600 Saarbrücken (DE)**

(72) Erfinder: **Härle, Anton, Dr., Schelmenstiege 8, D-4400 Münster-Roxel (DE)**

(74) Vertreter: **Habbel, Hans-Georg, Dipl.-Ing., Postfach 3429 Am Kanonengraben 11, D-4400 Münster (DE)**

Saugflasche mit einem in ein Operationsfeld einlegbaren Drain

Die Erfindung bezieht sich auf eine Saugflasche mit einem in ein Operationsfeld einlegbaren Drain, wobei an der Saugflasche ein mit einem öffenbaren Verschluß versehenes Verbindungsstück zum Anschluß eines Verbindungsschlauches zum Drain vorgesehen ist.

· Die vorerwähnten Teile bilden im ganzen eine Drainageeinrichtung, die zum Absaugen von Wundsekreten aus Körperhöhlen dient.

Bekannterweise wird bei einem notwendigen Wechsel der üblicherweise als Saugeinrichtung verwendeten Saugflasche zunächst der Verschluß des Verbindungsstückes geschlossen. Dann wird der Verbindungsschlauch von der Saugflasche getrennt und anschließend eine neue Saugflasche angeschlossen. Durch Öffnen des an der neuen Saugflasche vorhandenen Verschlusses wird der Absaugvorgang fortgesetzt.

Beim Wechseln der Saugflasche entfällt der Unterdruck oder Saugdruck in dem Verbindungsschlauch, so daß Sekrete, die sich im Drain und im Verbindungsschlauch befinden und die abgeführt werden sollten, zumindest teilweise in das Wundgebiet zurückfließen bzw. auch gegebenenfalls durch den im Wundgebiet noch herrschenden Unterdruck angesaugt werden.

Dadurch ist ein sehr großes Infektionsrisiko gegeben, durch das der Genesungs- und Heilungsprozeß nachteilig beeinflußt werden kann.

In der Praxis hat es sich gezeigt, daß Bakterien beim Abtrennen des Verbindungsschlauches von der Saugflasche z. B. 40 cm im Verbindungsschlauch in Richtung zu dem Wundgebiet vordringen und sich später auch gegen den Sekretstrom fortbewegend dem Drain und dem Wundgebiet zuwenden. Nach vergleichsweise kurzer Zeit ist dadurch eine sehr hohe Keimbesiedlung im Wundgebiet zu verzeichnen.

Aus der US-A-3 809 087 ist zwar eine Saugeinrichtung bekanntgeworden, bei der der Verbindungsschlauch mit einem Rückschlagventil ausgerüstet ist, das bei Aufheben des Unterdruckes an der Saugeinrichtung den Verbindungsschlauch schließt. Diese bekannte Einrichtung ist aber nicht mit den vom Patienten selbst zu tragenden Saugflaschenanordnungen vergleichbar, wobei ein Rückschlagventil im übrigen auch nicht die notwendige Sicherheit darstellt, da die Sitzfläche oder die Ventilkugel stets durch Blutgerinsel, Eiterkörperchen od. dgl. verschmutzt sein kann.

Der Erfindung liegt die Aufgabe zugrunde, die beispielsweise aus dem DE-U-7 336 232 bekannten Saugflaschen dahingehend zu verbessern, daß ein mit einer Drainage belegtes Wundgebiet auch nach mehrmaligem Saugflaschenwechsel weitgehend keimfrei zu halten bzw. die Keimbesiedlung zumindest wesentlich verringert werden kann.

Diese der Erfindung zugrundeliegende Aufgabe wird durch die Merkmale des kennzeichnenden Teiles des Hauptanspruches gelöst.

Beim Wechsel der Saugflasche kann somit der Verbindungsschlauch zunächst dicht verschlossen werden, so daß beim anschließenden Trennen der Saugflasche von dem Verbindungsschlauch ein Zurückströmen des in dem Drain und in dem Verbindungsschlauch befindlichen Sekretes vermieden werden kann. Über diese Vorteile hinaus wird durch die erfindungsgemäße Anordnung erreicht, daß nach dem Anschließen der neuen Saugflasche direkt abzusaugendes Sekret ansteht, so daß ein Ansaugen von Luft, wie es bei üblichen Verbindungsschläuchen zwangsläufig der Fall ist, vermieden werden kann. Das wirksame Saugvolumen der Saugflasche kann dadurch vergrößert und außerdem eine Kontrolle der Wundverhältnisse besser durchgeführt werden.

Aus der FR-A1-2 304 360 ist zwar eine Saugvorrichtung für Draineinrichtungen bekanntgeworden, bei der aber im Gegensatz zum Anwendungsgebiet der vorliegenden Anmeldung Spezialunterdruckflaschen eingesetzt werden müssen, die einen durchstechbaren Bereich aufweisen, wobei weiterhin der Verbindungsschlauch an seinem der Unterdruckflasche zugewandten Ende mit einer Spezialdurchstechnadel ausgerüstet sein muß.

Außerdem ist die Schiebeklemme auf dem Verbindungsschlauch ohne Zwischenschaltung eines Zwischenstückes angeordnet, so daß entweder, wenn der Verbindungsschlauch die erforderliche Starrheit in Abhängigkeit des in ihm wirkenden Unterdruckes aufweist, ein vollständiges Verschließen durch die Klemme nicht möglich ist oder aber der Verbindungsschlauch ist so elastisch gestaltet, daß er sich auch unter der Wirkung höherer Unterdrücke in der Saugflasche zusammenzieht.

Nachstehend ist die Erfindung anhand der Zeichnungen näher beschrieben.

Es zeigt

Fig. 1 eine Drainageeinrichtung mit einer Saugflasche, einem Verbindungsschlauch sowie einem Drain,

Fig. 2 eine im Längsschnitt gehaltene Absperrvorrichtung mit eingesteckten Verbindungsschlauchenden und

Fig. 3 eine Seitenansicht einer Schiebeklemme.

Eine im ganzen mit 1 bezeichnete Drainageeinrichtung zum Absaugen von Wundsekreten aus Körperhöhlungen weist eine Saugflasche 2, einen Verbindungsschlauch 3 sowie einen Drain 4 auf. Der Verbindungsschlauch 3 ist über ein Verbindungsstück 5 einerseits mit der Saugflasche 2 und am anderen Ende über ein Verbindungsteil 6 mit dem Drain 4 verbunden. In der Saugflasche 2 herrscht Unterdruck, so daß Wundsekret über den Drain 4 sowie den Verbindungsschlauch 3 in die Saugflasche 2 gefördert wird.

Das Verbindungsstück 5 weist einen Verschluß 7 auf, mit dem die Saugflasche 2 vor dem Anschließen des Verbindungsschlauches 3 dicht verschlossen wird, um den in der Saugflasche 2 herrschenden sterilen Zustand und den dort herrschenden Unterdruck aufrecht zu erhalten. Nach dem Einsetzen des Verbindungsschlauches 3 in das Verbindungsstück 5 kann der Verschluß 7 geöffnet werden.

Erfindungsgemäß weist nun auch der Verbindungsschlauch 3 zusätzlich eine Absperrvorrichtung 8 zum dichten Verschließen seines Durchtrittskanales 9 auf. Bei einem Wechsel der Saugflasche 2 kann dadurch der Verbindungsschlauch 3 geschlossen werden, so daß in vorteilhafter Weise beim Trennen des Verbindungsschlauches 3 von dem Verbindungsstück 5 das als Flüssigkeitssäule in dem Verbindungsschlauch 3 stehende Wundsekret nicht zu der Ansaugseite, d. h. zu dem Drain 4 und damit in das Wundgebiet zurückfließt.

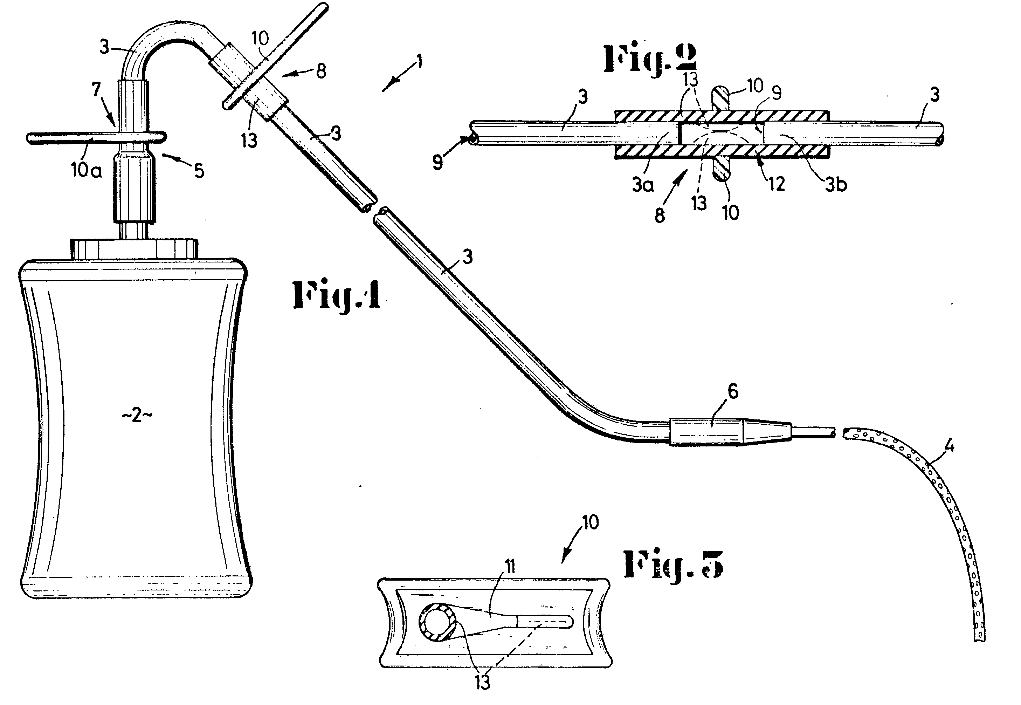

Die Absperrvorrichtung 8 ist, wie in Fig. 1 gut erkennbar, nahe an dem der Saugflasche 2 zugewandten Ende des Verbindungsschlauches 3 angeordnet.

Im dargestellten Ausführungsbeispiel weist die Absperrvorrichtung eine Schiebeklemme 10 auf. Auch der Verschluß 7 auf dem Verbindungsstück 5 ist mit einer solchen Schiebeklemme 10a ausgerüstet. Die Schiebeklemmen 10, 10a sind etwa plattenförmig ausgebildet (vgl. Fig. 2 und 3) und weisen einen sich in seiner Breite von einem zum anderen Ende hin verengenden Schlitz 11 auf (Fig. 3). Die jeweilige Schlitzbreite etwa an den Schlitzenden ist dabei derart bemessen, daß der durch diesen Schlitz geführte Schlauch od. dgl. in der einen Endstellung im wesentlichen in seinem Querschnitt unverändert bleibt, wie dies in Fig. 3 am linken Ende des Schlitzes 11 der Fall ist; andererseits kann der Schlauch in Schließstellung gebracht werden, wobei er in den engeren Teil des Schlitzes 11 eingeschoben wird. Seine Innenwandungen liegen dabei dicht aufeinander, wie dies strichliniert auch in Fig. 2 angedeutet ist.

Insbesondere Fig. 2 läßt gut erkennen, daß der Verbindungsschlauch 3 eine Unterbrechung aufweist, die durch ein die Absperrvorrichtung 8 enthaltendes Zwischenelement 12 überbrückt ist. Dieses Zwischenelement 12 ist im Ausführungsbeispiel durch ein gummielastisches Schlauchstück 13 gebildet, auf welchem die Schiebeklemme 10 der Absperrvorrichtung 8 angeordnet ist. Durch dieses gummielastische Schlauchstück 13 ist einerseits eine leichte Betätigung der Schiebeklemme 10 möglich und andererseits wird dadurch auch eine gute Dichtigkeit in Schließstellung der Absperrvorrichtung 8 erzielt.

Zweckmäßigerweise sind die beiden einander zugewandten Schlauchenden 3a, 3b und das als Zwischenelement 12 dienende Schlauchstück 13 wenigstens in einem Teil ihrer Überlappungsbereiche miteinander verklebt. Die Schlauchenden 3a, 3b sind dadurch einerseits in ihrer Lage gut fixiert und andererseits kann dadurch gegebenenfalls auch die Dichtigkeit im Überlappungsbereich verbessert sein.

Beim Wechsel der Saugflasche 2 wird nun folgendermaßen vorgegangen:

Der Absaugvorgang wird durch Verschieben der Schiebeklemme 10 in Schließstellung unterbrochen. Dabei wird das Schlauchstück 13 abgedrückt, wie es strichliniert in Fig. 2 und 3 erkennbar ist. Beim anschließenden Abziehen des Verbindungsschlauches 3 von dem Verbindungsstück 5 — gegebenenfalls nach Schließen der Schiebeklemme 10a — kann die in Saugrichtung vor der Absperrvorrichtung 8 anstehende Flüssigkeitssäule wegen der geschlossenen Absperrvorrichtung 8 nicht entgegen der Absaugrichtung abfließen.

Eine neue Saugflasche 2, in der Unterdruck herrscht und die zur Aufrechterhaltung dieses Unterdruckes mittels des Verschlusses 7 dicht verschlossen ist, kann dann mit dem freien Ende des Verbindungsschlauches 3 verbunden werden. Nach dem Öffnen zunächst des Verschlusses 7 an dem Verbindungsstück 5 und anschließendem Öffnen der Absperrvorrichtung 8 kann der Absaugvorgang fortgesetzt werden.

Neben dem besonderen Vorteil, daß durch die Ausbildung des Verbindungsschlauches 3 mit Absperrvorrichtung 8 ein Rückfließen des im Verbindungsschlauch sowie im Drain 4 befindlichen Sekretes vermieden wird, kann das zur Verfügung stehende Saugvolumen der Saugflasche 2 auch noch besser ausgenutzt werden. Bei dem Verbindungsschlauch 3 wird nämlich nach dem Öffnen des Verschlusses 7 sowie der Absperrvorrichtung 8 praktisch sofort Wundsekret gefördert; dagegen wird sonst wegen dem bei einem Saugflaschenwechsel zurückgeströmten Wundsekret zunächst als »Totvolumen« die in dem Schlauch befindliche Luft angesaugt. Dies kann sich insbesondere bei längeren Verbindungsschläuchen 3 nachteilig auswirken. Dagegen erfolgt bei dem erfindungsgemäßen Verbindungsschlauch praktisch ohne wesentliche Flüssigkeitsunterbrechung sofort eine Fortsetzung der Sekretförderung.

Insgesamt kann durch die neue Ausbildung einer Drainage das Infektionsrisiko, das insbesondere beim Saugflaschenwechsel besonders groß ist, wesentlich reduziert werden. Eine Keimbesiedlung des Wundgebietes durch zurückströmendes Wundsekret u. dgl. wird dabei vermieden.

## Patentanspruch

Saugflasche (2) mit einem in ein Operationsfeld einlegbaren Drain (4), wobei an der Saugflasche (2) ein mit einem öffenbaren Verschluß (7) versehenes Verbindungsstück (5) zum Anschluß eines Verbindungsschlauches (3) zum Drain (4) vorgesehen ist, dadurch gekennzeichnet, daß der Verbindungsschlauch (3) nahe an seinem der Saugflasche (2) zugewandten Ende ein Zwischenelement (12) aufweist, das durch ein insbesondere gummielastisches Schlauchstück (13) gebildet ist, auf welchem eine Schiebeklemme (10) als Absperrvorrichtung (8) zum dichten Verschließen seines Durchtrittskanales (9) angeordnet ist.

## Claim

A suction bottle (2) with a drain (4) which may be inserted in a field of operation, there being on the suction bottle (2) a connecting piece (5), which includes an openable closure device (7), for joining a connecting tube (3) to the drain (4), characterised in that the connecting tube (3) is provided with an intermediate element (12) close to the end next to the suction bottle (2), which element is formed particularly by a rubber-elastic tube piece (13), upon which there is arranged a sliding clip (10) as a shut-off device (8) for the tight closure of its internal channel (9).

## Revendication

Flacon à vide (2) comprenant un drain (4) insérable dans un champ opératoire, un raccord (5) muni d'un obturateur ouvrable (7) étant prévu pour raccorder un tube de liaison (3) du drain (4) au flacon à vide (2), caractérisé en ce que le tube (3) comprend à proximité de son extrémité d'amenée au flacon à vide (2) un élément intermédiaire (12) qui est formé par un morceau de tube (13) notamment en caoutchouc élastique sur lequel est disposé un tiroir coulissant (10), comme dispositif de blocage (8), pour fermer hermétiquement son canal traversier (9).

Fig.1

Fig.2

Fig.5

0 009 233